# DEMANDE DE BREVET EUROPEEN

(11) **EP 4 768 013 A1**
(43) Date de publication de la demande: **01.07.2026**
(21) Numéro de dépôt: 25307247.4
(22) Date de dépôt: 22.12.2025
(51) Int. Cl.: A61K 8/49, A61K 8/67, A61Q 19/08, A61Q 17/04, A61K 8/9771

(54) **COMPOSITION DERMATOLOGIQUE COMPRENANT DE L'EGCG ET DE L'ACIDE ASCORBIQUE**

(30) Priorité: 27.12.2024 BE 202405950
(71) Demandeur: Alphascience, 75008 Paris (FR)
(72) Inventeur: Marchal, Alfred, 1410 Waterloo (BE)
(74) Mandataire: Gevers & Orès

(57) **Abrégé**

La présente invention se rapporte à une composition dermatologique sous une forme essentiellement liquide, en particulier sous la forme d'un sérum, comprenant de l'épigallocatéchine gallate (EGCG) et de l'acide ascorbique.

## Description

La présente invention se rapporte à une composition dermatologique sous une forme essentiellement liquide, en particulier sous la forme d'un sérum, comprenant de l'épigallocatéchine gallate (EGCG) et de l'acide ascorbique.

L'apparence de la peau et les traitements de la peau font l'objet d'améliorations constantes dans le but de prévenir le vieillissement cutané. Le vieillissement de la peau connaît d'une part une cause intrinsèque liée à la dégradation des fonctions physiologiques avec le temps et d'autre part une cause extrinsèque liée aux agressions extérieures. De telles agressions proviennent de facteurs environnementaux tels que l'exposition aux UV (en particulier les UVA, les UVB et les UVC), aux infrarouges (IR) et aux autres éléments du spectre solaire, la pollution atmosphérique, ou une malnutrition. Afin de prévenir les imperfections cutanées, en particulier les imperfections au niveau du visage, des solutions sont développées pour protéger l'épiderme des agressions extérieures, pour stimuler la régénération de la peau et pour atténuer l'inflammation cutanée.

Dans le domaine des traitements de la peau, de nombreuses formulations cosmétiques reposent sur l'utilisation de substances actives permettant de protéger et de régénérer l'épiderme. Parmi ces substances actives, les catéchines, plus particulièrement l'épigallocatéchine gallate (EGCG), et l'acide ascorbique (ou vitamine C) sont des composés de choix mis en œuvre dans les compositions dermatologiques.

Les catéchines (dont l'EGCG) et l'acide ascorbique sont connus pour leurs effets dans le maintien et l'amélioration de l'apparence de l'épiderme. Leurs participations à la modulation des voies de signalisations cellulaires impliquées dans l'inflammation et la survie cellulaire, telles que MAPK et NF-kB, contribuent à la régénération cellulaire et à la réparation tissulaire. De plus, par leurs actions antioxydantes puissantes, l'EGCG et l'acide ascorbique neutralisent les radicaux libres et réduisent le stress oxydatif qui contribue au vieillissement cellulaire. Leurs propriétés anti-UV permettent aussi une protection optimale de la peau contre les agressions extérieures. En outre, l'EGCG et l'acide ascorbique stimulent la production de collagène, ce qui contribue à l'amélioration de l'élasticité de la peau. Une amélioration de ces différents paramètres permet globalement de protéger la peau contre les imperfections.

Des compositions dermatologiques comprenant de l'EGCG et de l'acide ascorbique sont connues et se présentent sous forme de crèmes puisque ces deux substances actives sont de façon inhérente instables en phase aqueuse. En effet, une fois dissous dans un solvant, en particulier dans de l'eau, l'EGCG et l'acide ascorbique sont sensibles à l'oxydation et se dégradent, ce qui ne rend pas possible la formulation de solutions aqueuses stables comprenant ses deux substances actives. Pourtant, en cosmétique et en particulier en dermatologie, l'utilisation de formulations sous forme liquide ou essentiellement liquide, comme par exemple l'utilisation d'un sérum, est largement préférée, notamment pour augmenter le coefficient de pénétration des actifs dans l'épiderme et améliorer ainsi leur efficacité.

Le document US2012177738 porte sur des compositions comprenant de l'EGCG, de l'acide ascorbique et un antioxydant de la famille des polyphénols, les stabilités de ces compositions étant évaluées au cours du temps. En particulier, il ressort de ce document antérieur que l'EGCG présente dans de telles compositions n'est pas stable au cours du temps.

Malheureusement, comme indiqué ci-dessus, les formulations dermatologiques actuelles comprenant de l'EGCG et de l'acide ascorbique présentent une stabilité insuffisante de l'EGCG au cours du temps même si ces formulations comprennent un antioxydant de la famille des polyphénols. Par ailleurs, les formulations dermatologiques actuelles comprenant de l'EGCG, de l'acide ascorbique et éventuellement un antioxydant de la famille des polyphénols se présentent en général sous forme de crèmes, ce qui limite considérablement la quantité d'actifs dans la composition et la pénétration des actifs dans l'épiderme. Par conséquent, les effets des actifs (par exemple les effets protecteur et régénérant) contenus dans les compositions actuelles, en particulier dans les crèmes, sont réduits au niveau des cellules de l'épiderme.

Il existe donc à ce jour un réel besoin de procurer une composition comprenant de l'EGCG et de l'acide ascorbique comme substances actives qui soit stable au cours du temps au moins en ce qui concerne l'EGCG et qui garantisse une pénétration optimale des actifs dans / au travers de l'épiderme.

Pour résoudre au moins en partie ces problèmes, il est prévu suivant l'invention, une composition dermatologique sous une forme essentiellement liquide, en particulier sous forme d'un sérum, comprenant :
- de l'épigallocatéchine gallate (EGCG),
- de l'acide ascorbique, et
- un extrait de Ginkgo biloba, en particulier un extrait de feuilles de Gingko biloba.

Au sens de la présente invention, l'extrait de Ginkgo biloba est présent notamment en tant qu'antioxydant additionnel, c'est-à-dire qu'il est présent notamment en tant qu'antioxydant en plus des actifs d'une composition selon l'invention étant des antioxydants, c'est-à-dire en plus de l'acide ascorbique et de l'EGCG. Au sens de la présente invention, un antioxydant additionnel est un antioxydant autre que les composés essentiels déjà compris dans la composition dermatologique selon l'invention, c'est-à-dire autre que l'EGCG et l'acide ascorbique.

L'EGCG est la catéchine la plus abondante et active du thé, notamment du thé vert, connue pour son puissant pouvoir antioxydant et ses propriétés anti-inflammatoires, protégeant les cellules, favorisant la perte de poids, la santé cardiovasculaire et ayant un potentiel dans la prévention du cancer en inhibant l'angiogenèse et en induisant l'apoptose cellulaire. On la trouve dans le thé vert, le thé blanc, et divers suppléments, agissant sur plusieurs voies cellulaires pour une action polyvalente.

L'acide ascorbique, mieux connu sous le nom de vitamine C, est une vitamine hydrosoluble essentielle, puissamment antioxydante, cruciale pour le système immunitaire, la synthèse du collagène (peau, tissus conjonctifs) et présente naturellement dans les agrumes et légumes.

Le Ginkgo biloba est un arbre dont les extraits, notamment les extraits de feuilles, sont largement utilisés en phytothérapie pour leurs bienfaits sur la circulation sanguine et les fonctions cognitives. Le Ginkgo biloba ne contient pas d'EGCG (épigallocatéchine gallate), ceci au contraire du thé, en particulier au contraire du thé vert (Camellia sinensis). En effet, le Ginkgo biloba et le thé, en particulier le thé vert, contiennent des substances actives distinctes qui leur confèrent des propriétés différentes :
- le Ginkgo biloba, en particulier les feuilles de Ginkgo biloba, contiennent principalement deux groupes de composés actifs : les flavonoïdes et les terpénolactones. Ces composés sont connus pour leurs effets antioxydants et pour améliorer la circulation sanguine,
- l'EGCG est le principal polyphénol et la catéchine la plus abondante dans l'extrait de thé, en particulier dans l'extrait de thé vert. Les propriétés antioxydantes puissantes de l'EGCG sont souvent associées aux bienfaits pour la santé du cœur et du cerveau, ainsi qu'à la gestion du poids.

Dans le cadre de la présente invention, il a été montré qu'une telle composition dermatologique suivant l'invention est stable au cours du temps même si la composition dermatologique se présente sous une forme essentiellement liquide, en particulier sous la forme d'un sérum, l'EGCG et l'acide ascorbique ne se dégradant pas ou ne se dégradant que de façon non significative au cours du temps. Plus particulièrement, il a été montré que la présence d'un extrait de Ginkgo biloba, dans une composition dermatologique comprenant de l'EGCG et l'acide ascorbique, permet de formuler une composition essentiellement liquide, par exemple de formuler un sérum, dans laquelle ces deux substances actives sont stables au cours du temps ou présentent du moins une stabilité augmentée au cours du temps, notamment par rapport à des compositions sous forme de crèmes comprenant ces deux mêmes substances actives ou par rapport à des compositions telles que celles décrites dans le document US2012177738.

En d'autres termes, une telle composition dermatologique selon la présente invention permet l'obtention d'une composition sous une forme essentiellement liquide ayant une stabilité augmentée dans le temps, en particulier en ce qui concerne l'EGCG et l'acide ascorbique, plus particulièrement en ce qui concerne l'EGCG, dont les stabilités sont augmentées au cours du temps. Il en découle qu'une composition dermatologique selon la présente invention permet d'assurer une conservation des propriétés dermatologiques de l'EGCG et de l'acide ascorbique, plus particulièrement de l'EGCG, sur une longue durée (d'au moins 2 mois) même si ces deux substances actives sont présentent dans une composition dermatologique se présentant sous une forme essentiellement liquide.

Par le terme « stabiliser », il est entendu que l'effet thérapeutique assuré par les actifs de la composition dermatologique est maintenu pendant toute la durée du stockage de ladite composition, ceci se traduisant par le maintien au cours du temps de la concentration de chacun des actifs dans la composition dermatologique.

Avec une telle composition dermatologique suivant l'invention, les problématiques mentionnées plus haut sont au moins partiellement solutionnées. En ce sens, la problématique de stabilité des actifs, tels que l'EGCG et l'acide ascorbique, dans une composition sous une forme essentiellement liquide, est résolue par une augmentation et un maintien de la stabilité des actifs au cours du temps. Par conséquent, une telle composition dermatologique sous une forme essentiellement liquide selon l'invention permet de conserver les effets bénéfiques conférés par les substances actives durant toute la durée de conservation de la composition dermatologique selon l'invention. En outre, avec une formulation/composition selon l'invention sous une forme essentiellement liquide, par exemple sous la forme d'un sérum, conférant une augmentation du coefficient de pénétration cutanée des substances actives, les effets bénéfiques des substances actives au niveau de l'épiderme sont augmentés, ceci permettant d'assurer une meilleure protection et une meilleure régénération de l'épiderme et de lutter plus efficacement contre le vieillissement cutané.

Selon l'invention, la composition dermatologique comprend de l'épigallocatéchine gallate (EGCG) et de l'acide ascorbique. Ces deux composés actifs ou substances actives sont des antioxydants puissants jouant un rôle protecteur contre l'environnement extérieur en réduisant le stress oxydatif induit par les radicaux libres. Ces deux composés actifs induisent également une protection contre la pollution, les UV, et la dénaturation de l'ADN. En particulier, la composition dermatologique selon l'invention est une composition anti-âge.

Par les termes « composition dermatologique », il est entendu au sens de la présente invention, une composition destinée à être utilisée par administration topique, de préférence sur la peau, les cheveux, les ongles ou les muqueuses. Une telle composition comprenant une quantité dermatologiquement active d'EGCG et d'acide ascorbique, permet d'obtenir un effet augmenté de l'EGCG et de l'acide ascorbique localement au niveau de la zone d'administration. Au sens de l'invention, ladite composition dermatologique participe à la diminution des effets du vieillissement, plus particulièrement, en réduisant le processus de vieillissement cutané tel que le vieillissement cellulaire en partie dû au stress oxydatif et aux agressions extérieures comme par exemple une exposition aux UV (en particulier les UVA, les UVB et les UVC), aux infrarouges (IR) et aux autres éléments du spectre solaire.

Par les termes « composition dermatologique sous une forme essentiellement liquide », il est entendu au sens de la présente invention, une composition ayant une faible densité et une faible viscosité, en particulier une composition essentiellement aqueuse ou aqueuse dans laquelle les composés de la composition dermatologique sont solubilisés / dissous. Une composition dermatologique sous une forme essentiellement liquide, en comparaison avec une composition dermatologique sous forme d'une crème ou d'un onguent, présente avantageusement un coefficient de pénétration cutanée (pénétration transcutanée) des substances actives qui est significativement augmenté au niveau des zones d'administration. Un meilleur coefficient de pénétration cutanée permet d'augmenter l'absorption cutanée des principes actifs et par conséquent d'augmenter l'efficacité du traitement par exemple contre le vieillissement cutané. Typiquement, les compositions dermatologiques sous une forme essentiellement liquide sont conditionnées sous la forme de bouteilles munies d'un compte-gouttes, d'un roll-on, ou d'une pompe.

Par le terme « extrait », lorsqu'il s'applique à un végétal ou à une partie d'un végétal, il est entendu selon l'invention, un extrait, en particulier un extrait sous forme d'un concentré, qui comprend la majorité voire l'ensemble des molécules et donc des principes actifs du végétal ou de la partie du végétal. De préférence, un tel extrait garantit une concentration standardisée des composés utiles/des principes actifs retrouvés dans le végétal ou dans une partie du végétal. Préférentiellement, l'extrait comprend les mêmes actifs dans des proportions relativement similaires voire dans des proportions similaires à celles observées pour dans le végétal ou dans une partie du végétal. Préférentiellement, l'extrait permet de maintenir les ratios tels qu'observés dans le végétal ou dans une partie du végétal entre la plupart voire entre toutes les molécules présentes dans le végétal ou dans une partie du végétal.

Avantageusement, selon un mode de réalisation suivant l'invention, l'extrait de Ginkgo biloba est un extrait naturel obtenu au départ de la totalité du végétal Ginkgo biloba ou d'une partie ou de plusieurs parties du végétal Ginkgo biloba, par exemple au départ de racines, et/ou de feuilles, et/ou de l'écorce, et/ou de toute autre partie de Ginkgo biloba.

Avantageusement, selon un mode de réalisation suivant l'invention, l'extrait de Ginkgo biloba est un extrait de feuilles de Ginkgo biloba. Dans le contexte de la présente invention, le terme « extrait de feuilles de Ginkgo biloba » correspond à sa signification usuelle conne dans l'état de la technique. En général, un extrait de feuilles de Ginkgo biloba peut être obtenu par extraction à l'aide d'un solvent organique (comme le propylène glycol et/ou l'éthanol) à partir de feuilles de Ginkgo biloba. Le processus d'extraction peut également contenir des étapes additionnelles. Généralement, les extraits de feuilles de Ginkgo biloba sont riches en flavones glycosides et en terpènes lactones, et contiennent des bilobalides, des ginkgolides (A, B et C), de la quercétine et du camphérol.

Les extraits sont généralement obtenus par extraction à l'aide d'un solvent et sont ensuite concentrés et/ou séchés. Donc, dans le contexte de la présente invention, le terme « extrait » comprend à la fois les extraits solides et liquides. De préférence, l'extrait de feuilles de Ginkgo biloba est un extrait liquide.

De préférence, selon l'invention, l'extrait de Ginkgo biloba comprend au moins l'un des antioxydants suivants de la famille des polyphénols : le camphérol, la quercétine, la rutine, l'isorhamnétine, les catéchines, les polyphénols de la famille des phlorotanins, et leurs mélanges.

Avantageusement, ladite composition selon l'invention comprend, relatif au poids total de la composition, au moins 10 % en poids, de manière encore plus avantageuse au moins 20 % en poids, encore plus avantageusement au moins 25 % en poids, encore plus avantageusement au moins 30 % en poids, encore plus avantageusement au moins 35 % en poids d'un extrait de Ginkgo biloba, en particulier d'un extrait de feuilles de Ginkgo biloba.

Selon une variante préférée de l'invention, ladite composition selon l'invention comprend, relatif au poids total de ladite composition, de 25 à 35 % en poids d'un extrait de Ginkgo biloba, en particulier d'un extrait de feuilles de Ginkgo biloba.

Avantageusement, selon l'invention, l'extrait de Ginkgo biloba, en particulier l'extrait de feuilles de Ginkgo biloba, est un extrait standardisé entre 20 et 30% de glycosides de flavones.

Préférentiellement, selon l'invention, l'extrait de Ginkgo biloba, en particulier l'extrait de feuilles de Ginkgo biloba, est un extrait standardisé entre 20 et 30% de glycosides de flavones et entre 5 et 10% de lactones terpéniques.

Avantageusement, selon l'invention, ledit extrait de Ginkgo biloba comprend entre 10 et 20%, préférentiellement entre 12 et 15% d'actifs de Ginkgo biloba (bilobalide, ginkgolide A, ginkgolide B, ginkgolide C, quercétine, camphérol, isorhamnétine, acide ginkgolique, rutine) et un glycol comme solvant.

Avec une composition dermatologique selon l'invention, l'EGCG et l'acide ascorbique présentent un coefficient de pénétration cutanée augmenté au niveau de l'épiderme lors d'une administration topique en comparaison avec une composition sous forme de crème ou d'un onguent comprenant ces deux mêmes actifs. Par conséquent, la forme essentiellement liquide de la composition dermatologique selon l'invention permet l'obtention d'un effet augmenté notamment pour la réparation cutanée, pour la protection contre le vieillissement cutané et pour une protection face aux agressions extérieures comme par exemple une exposition aux UV (en particulier les UVA, les UVB et les UVC), aux infrarouges (IR) et aux autres éléments du spectre solaire. Par ailleurs, contrairement aux crèmes ou aux onguents qui laissent généralement subsister un film gras sur la peau, une composition dermatologique sous une forme essentiellement liquide suivant l'invention permet d'effectuer une application plus agréable sans formation d'un film gras en surface de l'épiderme.

Avantageusement, selon l'invention, l'épigallocatéchine gallate (EGCG) est présente à raison d'au moins 1% en poids, de préférence à raison d'au moins 3% en poids, préférentiellement à raison d'au moins 4% en poids, plus préférentiellement à raison d'au moins 5% en poids, plus préférentiellement encore à raison d'au moins 6% en poids, par rapport au poids total de la composition dermatologique.

Avantageusement, selon l'invention, l'épigallocatéchine gallate (EGCG) est présente à raison de 3 à 5% en poids par rapport au poids total de la composition dermatologique.

Préférentiellement, selon l'invention, l'acide ascorbique est présent à raison d'au moins 1% en poids, de préférence à raison d'au moins 3% en poids, préférentiellement à raison d'au moins 4% en poids, plus préférentiellement à raison d'au moins 5% en poids, plus préférentiellement encore à raison d'au moins 10% en poids, par rapport au poids total de la composition dermatologique.

Préférentiellement, selon l'invention, l'acide ascorbique est présent à raison de 5 à 10% en poids par rapport au poids total de la composition dermatologique.

Avantageusement, selon un mode de réalisation suivant l'invention, l'épigallocatéchine gallate (EGCG) est présente dans un extrait végétal en particulier dans un extrait de thé, plus particulièrement dans un extrait de thé vert. Selon ce mode de réalisation, l'épigallocatéchine gallate (EGCG) est donc naturelle (d'origine naturelle).

Avantageusement, selon l'invention, ledit extrait végétal, en particulier ledit extrait de thé, plus particulièrement ledit extrait de thé vert, est un extrait végétal standardisé entre 60 et 80% de polyphenols.

De préférence, selon l'invention, ledit extrait végétal, en particulier ledit extrait de thé, plus particulièrement ledit extrait de thé vert, est un extrait végétal standardisé entre 60 et 80% de polyphenols et entre 50 et 75 % de catéchines dont au moins 25% d'EGCG, de préférence dont au moins 50% d'EGCG.

Préférentiellement, selon l'invention, l'extrait végétal dans lequel est présente l'épigallocatéchine gallate (EGCG) est un extrait de thé, en particulier un extrait de thé vert. De préférence, l'épigallocatéchine gallate (EGCG) est présente dans un extrait de thé vert.

De préférence, selon l'invention, ledit extrait végétal dans lequel est présente l'épigallocatéchine gallate (EGCG) contient au moins 25% en poids d'épigallocatéchine gallate (EGCG), de préférence au moins 30% en poids d'épigallocatéchine gallate (EGCG), préférentiellement au moins 40% en poids d'épigallocatéchine gallate (EGCG), plus préférentiellement au moins 50% en poids d'épigallocatéchine gallate (EGCG), par rapport au poids total dudit extrait végétal.

Selon un autre mode de réalisation suivant l'invention, l'épigallocatéchine gallate (EGCG) est synthétique.

Par les termes « d'origine naturelle », il est entendu au sens de la présente invention, des composés extraits d'éléments issus de la nature / provenant de la nature.

Au sens de la présente invention, le terme « antioxydant » défini un agent qui ralentit ou empêche l'oxydation. Un agent antioxydant diminue notamment l'oxydation en captant les radicaux libres qui sont responsables du stress oxydatif et par conséquent qui sont responsables du vieillissement cellulaire.

Suivant la présente invention, l'EGCG peut être extrait de thé, en particulier de thé vert, et l'acide L-ascorbique peut être extrait d'un agrume.

Dans son état naturel, l'EGCG est principalement présente dans le thé vert, le thé oolong, le thé blanc ou le thé noir, ainsi que dans les fruits ou les fruits à coque, comme par exemple les canneberges, les fraises, les mûres, les kiwis, les cerises, les poires, les pêches, les pommes et le avocats, ou encore les pacanes, les pistaches et les noisettes.

L'acide ascorbique peut provenir d'une source naturelle car il est présent dans les végétaux de manière générale, et plus particulièrement dans certains agrumes tel que le citron, l'orange et le pamplemousse.

Selon un mode de réalisation suivant l'invention, l'acide ascorbique est l'acide L-ascorbique qui représente la forme naturelle de la vitamine C.

De préférence, la composition dermatologique selon l'invention comprend en outre de l'acide phytique comme antioxydant supplémentaire. L'acide phytique ayant des propriétés antioxydantes, sa présence potentielle dans la composition dermatologique suivant l'invention permet d'en augmenter plus encore la stabilité dans le temps.

De préférence, selon l'invention, l'acide phytique est présent à raison d'au moins 0,5% en poids, préférentiellement à raison d'au moins 1% en poids, encore plus préférentiellement à raison d'au moins 1,5% en poids, de manière préférée à raison d'au moins 2% en poids, par rapport au poids total de la composition dermatologique.

Préférentiellement, la composition dermatologique selon l'invention comprend en outre au moins un solvant choisi parmi l'eau, le 1,2-propanediol, le 1,3-propanediol, l'isopentyldiol, et autres diols et/ou polyols aliphatiques de C3 à C5, ainsi que leurs mélanges.

Selon un mode de réalisation préféré suivant l'invention, ledit au moins un solvant est le 1,3-propanediol. L'utilisation d'un tel solvant dans une composition dermatologique suivant l'invention est avantageuse en ce sens qu'il s'agit d'un solvant d'origine naturelle (« solvant vert ») et non pas d'un solvant synthétique tel que le 1,2-propanediol. En outre, la présence 1,3 propanediol, plutôt que la présence du 1,2-propanediol, dans la composition dermatologique selon l'invention permet l'obtention d'une composition dermatologique tout aussi stable.

Au sens de la présente invention, le solvant est un composé aqueux permettant de transporter de manière optimale les substances actives dans une forme adéquate afin que leurs propriétés actives soient au moins préservées et au mieux augmentées mais jamais diminuées.

De préférence, la composition dermatologique selon l'invention comprend en outre un ou plusieurs facteurs de pénétration cutanée et/ou un ou plusieurs agents humectant et/ou un ou plusieurs surfactants.

A titre d'exemple, peut être utilisé en tant que facteur de pénétration cutanée ou comme vecteur transcutané le transcutol. Un tel facteur de pénétration cutanée permet aux actifs d'une composition dermatologique de traverser la barrière de la peau ou l'épiderme d'autant plus facilement pour atteindre une cible, par exemple une cible thérapeutique.

A titre d'exemple, peut être utilisé en tant qu'agent humectant, le EG-1 liponic^{®}. Un tel agent humectant permet de formuler la composition dermatologique dans des conditions optimales pour la préservation des actifs. Aussi, un tel agent humectant permet d'assurer une hydratation de la peau / de l'épiderme améliorée.

A titre d'exemple, peut être utilisé en tant que surfactant le diméthicone tel que le PEG-8 diméthicone. La présence d'un ou de plusieurs surfactants dans la composition dermatologique selon l'invention permet d'en améliorer la stabilité et l'homogénéité. Le surfactant peut aussi participer à la production d'une composition dermatologique qui améliore la pénétration des principes actifs et la sensation de confort lors de l'application cutanée.

Avantageusement, la composition dermatologique selon l'invention présente un pH inférieur à 7, préférentiellement un pH inférieur à 6, plus préférentiellement un pH inférieur à 5, encore plus préférentiellement un pH inférieur à 4, encore plus préférentiellement un pH égal à 3,5.

De façon préférée, le pH d'une composition dermatologique selon l'invention est égal à 3,5, ce qui permet d'observer une pénétration transcutanée améliorée de l'acide ascorbique.

Préférentiellement, la composition dermatologique selon l'invention se présente sous la forme d'une solution, d'un sérum, d'une lotion, d'un aérosol, d'une pulvérisation, d'une dispersion ou d'un gel essentiellement liquide.

Selon un mode de réalisation préféré suivant l'invention, la composition dermatologique se présente sous la forme d'un sérum. Les sérums sont définis comme étant des formulations essentiellement liquides dans lesquelles les actifs sont présents à des concentrations plus élevées que celles observées pour les autres types de formulations. Ainsi, lorsque la composition dermatologique selon l'invention est sous la forme de sérum, l'effet des actifs est augmenté au niveau de la zone d'application en comparaison avec d'autres types de compositions dermatologiques. Le sérum est avantageux pour obtenir une efficacité maximale des substances actives de la composition dermatologique.

Selon un autre mode de réalisation suivant l'invention, la composition dermatologique selon l'invention est sous la forme d'une lotion. Une composition dermatologique sous la forme de lotion est moins concentrée en substances actives qu'une composition dermatologique sous la forme d'un sérum.

Selon encore un autre mode de réalisation suivant l'invention, la composition dermatologique selon l'invention est sous la forme d'un gel essentiellement liquide. Une composition dermatologique sous la forme d'un gel essentiellement liquide est moins concentrée en substances actives qu'une composition dermatologique sous la forme d'un sérum.

De préférence, la densité d'une composition dermatologique selon l'invention est comprise entre 1 et 1,1, de préférence est égale à 1,05.

De préférence, la composition dermatologique selon l'invention est destinée à une administration topique, de préférence sur la peau et/ou les cheveux.

La présente invention porte encore sur une utilisation d'une composition suivant l'invention comme médicament.

Préférentiellement, une composition dermatologique suivant l'invention est utilisée comme médicament dans le traitement ou la prévention des dommages causés par les rayons ultraviolets, en particulier par les UVA, les UVB et les UVC, par les rayons infrarouges et/ou par les autres éléments du spectre solaire, d'un trouble provoquant des lésions cutanées, d'un trouble provoquant un vieillissement cutané précoce, d'un trouble provoquant une inflammation cutanée, et/ou d'un trouble provocant une altération / une dénaturation de l'ADN, du collagène et/ou de la matrice extracellulaire (MEC).

Une utilisation comme médicament d'une composition dermatologique suivant l'invention assure notamment le traitement ou la prévention de troubles cutanés tels que des lésions cutanées, une inflammation cutanée, un vieillissement cutané mais aussi le traitement ou la prévention des effets mutagènes des rayons UV, des rayons **IR** et des autres éléments du spectre solaire par protection des cellules de l'épiderme face aux lésions et aux modifications induites par les rayons ultraviolets, en particulier les UVA, UVB et UVC, sur l'acide désoxyribonucléique (ADN).

Les troubles provoquant des lésions cutanés ou une inflammation cutanée au sens de la présente invention sont par exemple, l'acné, le psoriasis, la dermatite atopique, ou la rosacée. Les troubles provoquant un vieillissement cutané précoce sont par exemple la maladie de Gottron, le syndrome de Werner, ou la maladie de Hutchinson-Gilford.

Les dommages causés par les rayons ultraviolets sont notamment une distension de la peau et un vieillissement cellulaire.

La présente invention porte encore sur une utilisation cosmétique non-thérapeutique de la composition dermatologique selon l'invention, pour favoriser la densification et/ou l'homogénéisation de l'épiderme, atténuer les rides, repulper et/ou raffermir la peau, pour protéger la peau des dommages causés par les rayons ultraviolets, en particulier par les UVA, les UVB et les UVC, par les rayons infrarouges et/ou par les autres éléments du spectre solaire, pour protéger la peau d'un trouble provoquant des lésions cutanées, d'un trouble provoquant un vieillissement cutané précoce et/ou d'un trouble provoquant une inflammation cutanée et/ou pour protéger l'ADN, le collagène et/ou la matrice extracellulaire (MEC).

De préférence, suivant l'invention, l'utilisation cosmétique non-thérapeutique de la composition dermatologique selon l'invention est topique, de préférence sur la peau et/ou les cheveux.

D'autres caractéristiques, détails et avantages de l'invention ressortiront des exemples donnés ci-après, à titre non limitatif.

### Exemples

### Exemple 1 : Préparation d'une composition dermatologique selon l'invention

Une composition dermatologique selon l'invention, sous la forme d'un sérum dont le pH est égal à 3,67, a été préparée en mélangeant les différents composés suivants (pourcentage en poids relatif au poids total de la composition) : EGCG (1 à 5%), acide ascorbique (5 à 10%), acide phytique (0,5 à 2%), extrait de Ginkgo biloba standardisé entre 20 et 30% de glycosides de flavones et entre 5 et 10% de lactones terpéniques (25 à 35%), eau (1 à 73,5%), 1,3 propanediol (15 à 20%), aminométhyl propanol (AMP) (0,5 à 2%), diméthicone (0,5 à 2%), liponic EG-1^{®} (0,4%), transcutol (1 à 3%), isopentyldiol (20 à 25%).

### Exemple 2: Evaluation de la stabilité des substances actives d'une composition dermatologique selon l'invention

La stabilité de la composition dermatologique préparée selon l'exemple 1 a été évaluée sur une période de 2 mois, pour l'EGCG et l'acide L-ascorbique.

Pour l'EGCG, une courbe de calibration a été réalisée en dissolvant une solution initiale comprenant une concentration de 20mg/mL d'EGCG dans une solution de 70 : 30 (v/v) eau : méthanol pour obtenir des concentrations de 0,1, 0,2, 1,0 et 2,0 mg/mL d'EGCG.

Pour l'acide L-ascorbique, une courbe de calibration a été réalisée en dissolvant une solution initiale comprenant une concentration de 20mg/mL de L-acide ascorbique dans une solution de 50 : 50 (v/v) eau : acétonitrile pour obtenir des concentrations de 0,1, 0,2, 1,0 et 2,0 mg/mL d'acide L-ascorbique.

Chacune des courbes de calibrations pour l'analyse de l'EGCG et pour l'analyse de l'acide L-ascorbique a été réalisée à partir de quatre points en rapportant les aires sous les pics mesurés en HPLC à 275 nm en fonction de la masse d'EGCG ou à 254 nm en fonction de la masse d'acide L-ascorbique injectée sur la colonne HPLC et des concentrations des standards respectivement. Chaque solution standard a été analysée trois fois en HPLC afin de réaliser une moyenne sur les intégrations de l'aire sous le signal correspondant à l'EGCG ou à l'acide L-ascorbique, respectivement.

Pour l'analyse de l'EGCG, un échantillon de la composition dermatologique de 163,1 mg a été pesé dans un ballon de 10 mL et ensuite dilué dans 5 mL d'une solution de 70 : 30 (v/v) eau : méthanol. La solution aqueuse est centrifugée 10 minutes à 4,500 rpm. Finalement, 1 mL de la solution centrifugée est collectée pour l'analyse HPLC.

Pour l'analyse de l'acide L-ascorbique, un échantillon de la composition dermatologique de 57,9 mg a été pesé dans un ballon de 10 mL et ensuite dilué dans 5 mL d'une solution de 50 : 50 (v/v) eau : acétonitrile. La solution est centrifugée 10 minutes à 4,500 rpm. Finalement, 1 mL de la solution centrifugée est collectée pour l'analyse HPLC.

Les conditions de l'analyse sont les suivantes :
- Appareillage : HPLC Agilent 1100 series connecté à un détecteur UV-Vis.
- Colonne : Agilent Eclipse Plus C18 (3,5 µm) - 4,6 x 100 mm
- Flux :
   ∘ pour l'analyse de l'EGCG : 1,2 mL/min,
   ∘ pour acide ascorbique : 1,0 mL/min
- Volume d'injection : 2 µL
- Eluant :
   ∘ Pour l'analyse de l'EGCG : la phase mobile est une solution composée de 70 : 30 (v/v) eau : méthanol.
   ∘ Pour l'analyse de l'acide ascorbique : la phase mobile est une solution composée de 50 : 50 (v/v) solution aqueuse (4mM) de NaHpSO contenant 0,1% en poids de HCOOH : acétonitrile.
- Température colonne : 40°C
- Longueurs d'onde sélectionnées :
   ∘ Pour l'analyse de l'EGCG : 275 nm
   ∘ Pour l'analyse de l'acide L-ascorbique : 254 nm

La teneur en EGCG ou la teneur en acide L-ascorbique dans la composition dermatologique a été évaluée sur une période de 2 mois. A cet effet, la quantité d'EGCG d'une part et la quantité d'acide L-ascorbique d'autre part ont été dosée à intervalles réguliers. La stabilité a été évaluée dans des conditions de conservation normales (20-25°c, HR = 60%, à l'obscurité).

Les résultats de ces expériences sont présentés dans le Tableau 1 ci-dessous.

**Tableau 1**

| **Durée de conservation (mois)** | **Dosage de l'EGCG (% en poids relatif au poids total de la composition dermatologique)** | **Dosage de l'acide L-ascorbique (% en poids relatif au poids total de la composition dermatologique)** |
|---|---|---|
| 0 | 3,86 | 8,62 |
| 2 | 3,82 | 8,50 |

Les résultats obtenus montrent que la composition dermatologique selon l'invention est stable au cours du temps tant pour l'EGCG que pour l'acide ascorbique puisque 98,9% d'EGCG et 98,6% d'acide ascorbique sont toujours présents dans la composition selon l'invention après 2 mois de conservation par rapport aux quantités initialement présentes. Par ailleurs, sur l'ensemble de la durée du test aucune altération de la coloration ni de l'odeur de la composition dermatologique n'a pu être observée. Ces observations sont surprenantes étant donné que l'EGCG et l'acide ascorbique sont généralement oxydés en quelques heures dans des formulations cosmétiques de ce type, c'est-à-dire dans des formulations cosmétiques essentiellement liquides.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition dermatologique sous une forme essentiellement liquide, en particulier sous forme d'un sérum, comprenant :
- de l'épigallocatéchine gallate (EGCG),
- de l'acide ascorbique, et
- un extrait de Gingko biloba, en particulier un extrait de feuilles de Gingko biloba.

2. Composition dermatologique selon la revendication 1, **caractérisée en ce que** l'épigallocatéchine gallate (EGCG) est présente à raison d'au moins 1% en poids, de préférence à raison d'au moins 3% en poids, préférentiellement à raison d'au moins 4% en poids, plus préférentiellement à raison d'au moins 5% en poids, plus préférentiellement encore à raison d'au moins 6% en poids, par rapport au poids total de la composition dermatologique.

3. Composition dermatologique selon la revendication 1 ou 2, **caractérisée en ce que** l'acide ascorbique est présent à raison d'au moins 1% en poids, de préférence à raison d'au moins 3% en poids, préférentiellement à raison d'au moins 4% en poids, plus préférentiellement à raison d'au moins 5% en poids, plus préférentiellement encore à raison d'au moins 10% en poids, par rapport au poids total de la composition dermatologique.

4. Composition dermatologique selon l'une quelconque des revendications précédentes, **caractérisée en ce que** ledit extrait de Gingko biloba, en particulier ledit extrait de feuilles de Gingko biloba, est présent à raison d'au moins 10% en poids, préférentiellement à raison d'au moins 20% en poids, plus préférentiellement à raison d'au moins 25% en poids, plus préférentiellement à raison d'au moins 30% en poids, plus préférentiellement encore à raison d'au moins 35% en poids, par rapport au poids total de la composition dermatologique.

5. Composition dermatologique selon l'une quelconque des revendications précédentes, **caractérisée en ce que** l'épigallocatéchine gallate (EGCG) est présente dans un extrait végétal, en particulier dans un extrait de thé, plus particulièrement dans un extrait de thé vert.

6. Composition dermatologique selon la revendication 5, **caractérisée en ce que** ledit extrait végétal dans lequel est présente l'épigallocatéchine gallate (EGCG) contient au moins 25% en poids d'épigallocatéchine gallate (EGCG), de préférence au moins 30% en poids d'épigallocatéchine gallate (EGCG), préférentiellement au moins 40% en poids d'épigallocatéchine gallate (EGCG), plus préférentiellement au moins 50% en poids d'épigallocatéchine gallate (EGCG), par rapport au poids total dudit extrait végétal.

7. Composition dermatologique selon l'une quelconque des revendications précédentes, **caractérisée en ce qu'**elle comprend en outre au moins un solvant choisi parmi l'eau, le 1,2-propanediol, le 1,3-propanediol, l'isopentyldiol, et autres diols et/ou polyols aliphatiques de C3 à C5, ainsi que leurs mélanges.

8. Composition dermatologique selon la revendication 8, **caractérisée en ce que** ledit au moins un solvant est le 1,3-propanediol.

9. Composition dermatologique selon l'une quelconque des revendications précédentes, **caractérisé en ce qu'**elle se présente sous la forme d'une solution, d'un sérum, d'une lotion, d'un aérosol, d'une pulvérisation, d'une dispersion ou d'un gel essentiellement liquide.

10. Composition dermatologique selon l'une quelconque des revendications précédentes étant destinée à une administration topique, de préférence sur la peau et/ou les cheveux.

11. Composition dermatologique selon l'une quelconque des revendications précédentes pour utilisation comme médicament.

12. Composition dermatologique selon la revendication 11, pour utilisation dans le traitement ou la prévention des dommages causés par les rayons ultraviolets, en particulier par les UVA, les UVB et les UVC, par les rayons infrarouges et/ou par les autres éléments du spectre solaire, d'un trouble provoquant des lésions cutanées, d'un trouble provoquant un vieillissement cutané précoce, d'un trouble provoquant une inflammation cutanée, et/ou d'un trouble provocant une altération / une dénaturation de l'ADN, du collagène et/ou de la matrice extracellulaire (MEC).

13. Utilisation cosmétique non-thérapeutique de la composition dermatologique selon l'une quelconque des revendications 1 à 9, pour favoriser la densification et/ou l'homogénéisation de l'épiderme, atténuer les rides, repulper et/ou raffermir la peau, pour protéger la peau des dommages causés par les rayons ultraviolets, en particulier par les UVA, les UVB et les UVC, par les rayons infrarouges et/ou par les autres éléments du spectre solaire, pour protéger la peau d'un trouble provoquant des lésions cutanées, d'un trouble provoquant un vieillissement cutané précoce et/ou d'un trouble provoquant une inflammation cutanée et/ou pour protéger l'ADN, le collagène et/ou la matrice extracellulaire (MEC).

14. Utilisation cosmétique non-thérapeutique selon la revendication 13, **caractérisée en ce qu'**elle est topique, de préférence sur la peau et/ou les cheveux.
